# EUROPEAN PATENT APPLICATION

(11) **EP 4 233 976 A1**
(43) Date of publication of application: **30.08.2023**
(21) Application number: 21890929.9
(22) Date of filing: 21.10.2021
(51) Int. Cl.: A61M 25/14

(54) **CATHETER AND CHOKE CATHETER**

(30) Priority: 13.11.2020 CN 202011272049
(71) Applicant: MicroPort NeuroTech (Shanghai) Co., Ltd., Shanghai 201318 (CN)
(72) Inventor: CUN, Yuxi, Shanghai 201318 (CN); LIU, Yunyun, Shanghai 201318 (CN); LIU, Yumei, Shanghai 201318 (CN); SUN, Li, Shanghai 201318 (CN)
(74) Representative: Marks & Clerk LLP
(86) International application number: PCT/CN2021/125444
(87) International publication number: WO 2022/100402

(57) **Abstract**

A catheter includes a tubular component (100) and a functional component. The tubular component (100) includes an outer tube (101) and an inner tube (102). The outer tube (101) is sleeved over the inner tube (102), and a first lumen is formed between the outer tube (101) and the inner tube (102). The outer tube (101) includes an outer tube main segment (1011) and a first recess (1012). The first recess (1012) is located at a distal end of the outer tube main segment (1011). The functional component is disposed on the tubular component (100) and is at least partially connected to the first recess (1012). This arrangement imparts increased compatibility and higher accessibility to the catheter with the functional component.

## Description

### TECHNICAL FIELD

The present invention relates to the field of medical devices and, in particular, to a catheter and an occlusion catheter.

### BACKGROUND

Strokes, mainly caused by blood clots in cerebral blood vessels, are a common medical condition that seriously threatens human health and also the third leading cause of death worldwide and the number one cause of long-term disability in adults. In the current clinical practice, a blocked blood vessel is often recanalized by removing the thrombus directly with an aspiration catheter or with the aid of a stent. After the aspiration catheter is passed through blood vessels to the blocked site, a negative pressure is applied at a proximal end of the aspiration catheter to suck the clot into the catheter or onto its end, followed by slow retraction of the clot into a guide catheter. As a result, the blood vessel recovers back to its normal hemodynamic condition. The stent-assisted thrombus removal approach involves passing the stent through the clot to trap the thrombus within struts of the stent and then retracting it back into a support catheter to recanalize the blood vessel. After the stent is pulled back into the support catheter, the latter is in turn withdrawn, together with the stent therein and the blood clot trapped in the stent, into a guide catheter. However, during the thrombus removal process, under the action of blood flow from the proximal end, break-off of the clot and subsequent migrating the break-off clot into a distal blood vessel has been frequently reported. It is also possible that the successfully trapped clot is fragmented into multiple pieces due to manipulation of the interventional instrument (the guide or support catheter) involved in the operation of the aspiration catheter or thrombectomy stent. Such clot fragments may flow with the blood up to a distal blood vessel and may lead to reocclusion there, which makes the surgical procedure unsuccessful, and may even threaten the patient's life in severe cases. For example, the possibility of percutaneous coronary intervention (PCI) caused myocardial necrosis reaches as high as 16%-39%, and most of these cases have been found to be attributable to escape of clots into distal blood vessels during the PCI procedures. Conventionally, the problem of clot fragmentation that may occur during an interventional procedure has been addressed usually by facilitating the thrombus removal operation through temporarily occluding the blood flow with a balloon guide catheter.

In a typical such procedure, after a thrombus removal element is delivered to a target site with the assistance of a balloon guide catheter, the balloon may be expanded against the blood vessel wall by injecting a contrast fluid therein, thus temporarily occlude blood flow in the vessel. Moreover, after the blood clot has been retrieved, the balloon is caused to contract, followed by withdrawal of the balloon guide catheter. In this way, the blood clot is taken out of the patient's body, and blood flow is recovered in the vessel. As a functional component attached to the catheter, the balloon tends to affect the overall compatibility and accessibility of the catheter. Apart from such balloon guide catheters, the same problem may also exist for other catheters with functional components such as occlusion, electronic, radiopaque and thrombus removal components.

Therefore, existing catheters with functional components are associated with the problem of degraded compatibility and accessibility.

### SUMMARY OF THE INVENTION

It is an object of the present invention to provide a catheter, which is free of the problem of degraded compatibility and accessibility as seen in conventional catheters.

To this end, the present invention provides a catheter comprising a tubular component and a functional component,
the tubular component comprising an outer tube and an inner tube,
the outer tube sleeved over the inner tube, a first lumen formed between the outer and inner tubes, the outer tube comprising an outer tube main segment and a first recess, the first recess located at a distal end of the outer tube main segment,
the functional component disposed on the tubular component and at least partially connected to the first recess.

Preferably, the functional component may be at least one of an occlusion component, a radiopaque component, an electronic element and a thrombus removal element.

Preferably, the first recess may be formed by an inward recess in an outer surface of the outer tube.

Preferably, an inner diameter of the first recess may be smaller than or equal to an inner diameter of the outer tube main segment.

Preferably, the first recess may be defined by an outward recess in an inner surface of the outer tube.

Preferably, an outer diameter of the first recess may be smaller than or equal to an outer diameter of the outer tube main segment.

Preferably, the first recess may have an axial length of 2-30 mm.

Preferably, the first recess may comprise a first transition section and a first straight section from a proximal end to a distal end thereof, the first transition section being a diameter-varying section where at least one of an inner diameter and an outer diameter of the outer tube increases or decreases.

Preferably, the first transition section may have an axial length of 0 mm to 10 mm.

Preferably, an inner surface and/or an outer surface of the first transition section may be each inclined at an angle of 0°-90° with respect to an axis of the outer tube main segment.

Preferably, the inner and outer surfaces of the first transition section may be inclined at a same angle with respect to the axis of the outer tube main segment.

Preferably, a ratio of an outer diameter of the first straight section to an outer diameter of the outer tube main segment may be 0.7-1.0.

Preferably, the outer diameter of the outer tube main segment may be 1.0 mm to 3.7 mm, and the outer diameter of the first straight section may be 0.7 mm to 3.5 mm.

Preferably, the outer tube may further comprise an outer-tube distal section located at a distal end of the first recess, an outer diameter of the outer-tube distal section at a proximal end thereof is greater than an outer diameter of the first recess at the distal end thereof, the outer-tube distal section fixedly connected to the inner tube at a distal location.

Preferably, the outer-tube distal section may comprise a second transition section and a second straight section from a proximal end to a distal end thereof, the second transition section being a diameter-varying section where at least one of an inner diameter and an outer diameter of the outer tube increases or decreases.

Preferably, the inner tube may comprise an inner tube main segment and a second recess, the second recess located at a distal end of the inner tube main segment, wherein the second recess is formed by an inward recess in an outer surface of the inner tube.

Preferably, the second recess may comprise a third transition section and a third straight section from a proximal end to a distal end thereof, the third transition section being a diameter-varying section where an outer diameter of the inner tube decreases.

Preferably, the second recess may have an axial length of 2-60 mm.

Preferably, an outer surface of the third transition section may be inclined at an angle of 0°-90° with respect to an axis of the inner tube main segment, and the third transition section may have an axial length of 0-10 mm.

Preferably, a ratio of an outer diameter of the third straight section to an outer diameter of the inner tube main segment may be greater than or equal to 0.6 and smaller than 1.0.

Preferably, the outer diameter of the inner tube main segment may be 0.5 mm to 3.2 mm, and the outer diameter of the third straight section may be greater than or equal to 0.3 mm and smaller than 3.2 mm.

Preferably, a most distal end of the outer tube main segment may form a first transition location, and a most distal end of the inner tube main segment may form a second transition location, wherein a projection of the first transition location on the axis of the tubular component is distal with respect to a projection of the second transition location on the axis of the tubular component;
the first recess comprises a first transition section and a first straight section from a proximal end to a distal thereof; and
an outer surface of the third transition section is inclined at a first angle with respect to the axis of the tubular component and an inner surface of the first transition section is inclined at a second angle with respect to the axis of the tubular component, the first angle being greater than or equal to the second angle.

Preferably, the inner tube further may comprise an inner-tube distal section located at a distal end of the second recess.

Preferably, the inner-tube distal section may have an axial length of 1-500 mm.

Preferably, an outer diameter of the inner-tube distal section may be smaller than an outer diameter of the second recess, and the inner-tube distal section may be disposed at a head portion of the catheter.

Preferably, the inner-tube distal section may comprise a proximal fourth transition section and a distal fourth straight section, the fourth transition section being a diameter-varying section where an outer diameter of the inner tube decreases.

Preferably, the fourth straight section may have an outer diameter of 0.2 mm to 3.1 mm.

Preferably, the functional component may be an occlusion component and be secured at a proximal end thereof to the first recess.

Preferably, the occlusion component may be a polymer film, wherein the occlusion component is in an expanded configuration when the first lumen is filled with a liquid, and wherein the occlusion component is in a collapsed configuration when the first lumen is in a vacuum.

Preferably, a distal end of the occlusion component may be fixedly connected to the inner tube.

Preferably, the occlusion component may be disposed on the first recess and each of a proximal end and the distal end of the occlusion component may be fixedly connected to the outer tube, wherein the outer tube is distally connected to the inner tube, and wherein a distal end of the first recess is provided with liquid passage apertures for dilation of the occlusion component with a liquid.

Preferably, the polymer film may have a thickness of 0.05 mm to 0.15 mm.

Preferably, the polymer film may be made of any one of silicone, polyurethane, latex, polyethylene, polytrafluoroethylene and expanded polytrafluoroethylene, or of a mixture thereof.

Preferably, the inner tube may comprise an inner tube main segment and a second recess, the second recess disposed at a distal end of the inner tube main segment, the second recess having an outer diameter smaller than an outer diameter of the inner tube main segment, wherein the functional component is an occlusion component, a proximal end of the occlusion component fixedly connected to the first recess and a distal end of the occlusion component fixedly connected to the second recess.

Preferably, each of the inner and outer tubes may comprise at least one polymer layer made of one or more of polyether block amide, nylon, polyurethane, polytrafluoroethylene, polyethylene and polyolefin elastomer.

Preferably, the outer tube and/or the inner tube may further comprise reinforcing layer(s), wherein the reinforcing layer is a structure braided from filaments, a structure consisting of spirally wound filaments, a cut tube or a combination thereof and is made of stainless steel, a nickel-titanium alloy, a cobalt-chromium alloy or a polymer.

Preferably, the outer tube and/or the inner tube may be triple-layered structure(s) each consisting of an innermost first polymer layer, an intermediate reinforcing layer and an outermost second polymer layer.

Preferably, the catheter may further comprise a first radiopaque ring disposed at a head portion of the catheter.

Preferably, the catheter may further comprise a second radiopaque ring disposed at a location on the inner tube, which is adapted to a location of the functional component.

Preferably, the inner tube may be provided with a second lumen having a constant inner diameter across its entire length.

Preferably, a ratio of the inner diameter of the second lumen to an outer diameter of the outer tube main segment may be 0.2-0.9.

Preferably, the inner diameter of the second lumen may be 0.1 mm to 3.0 mm, and the outer diameter of the outer tube main segment may be 0.5 mm to 3.7 mm.

Preferably, the first recess may have a tapered outer surface with a gradually decreasing outer diameter, wherein a proximal end of the functional component is secured to the tapered surface of the first recess.

Preferably, a distal end face of the outer tube may be a first beveled surface forming the outer surface of the first recess, wherein a proximal end of the functional component forms a second beveled surface mating with and fixedly connected to the first beveled surface.

The present invention also provides an occlusion catheter, comprising a tubular component and an occlusion component, the tubular component comprising an outer tube and an inner tube, the outer tube sleeved over the inner tube, a first lumen formed between the outer and inner tubes, the outer tube comprising an outer tube main segment and a first recess, the first recess located at a distal end of the outer tube main segment, the occlusion component disposed on the tubular component and at least partially connected to the first recess.

Preferably, the occlusion component may be a polymer film, wherein the occlusion component is in an expanded configuration when the first lumen is filled with a liquid, and wherein the occlusion component is in a collapsed configuration when the first lumen is in a vacuum.

In summary, the catheters of the present invention provide at least one of the following benefits:
1. The recess(es) provided in the outer tube and/or the inner tube of the catheter can accommodate at least a part of the functional component, allowing a reduced joint thickness, which can partially or completely eliminate the influence of the occlusion component on the catheter's hardness. In this way, it can be ensured that the catheter has sufficient flexibility that allows its smooth advancement through a blood vessel.
2. The recess(es) provided in the outer tube and/or the inner tube of the catheter allow(s) the catheter to have a reduced overall thickness, which enables the catheter to have a sufficiently large inner cavity but not an excessive outer diameter. Thus, relatively large medical devices can be delivered through the catheter's inner cavity, and the catheter itself can be smoothly advanced within a tortuous blood vessel while less stimulating the wall thereof to access a relatively distal location in the blood vessel.
3. Through securing a proximal end of the functional component to the outer tube and a distal end thereof to the inner tube, the influence of the presence of the functional component on the overall outer diameter and compliance of the catheter can be additionally reduced.
4. The recess provided in the inner tube ensures the lumen between the inner and outer tubes is large enough to perform its intended functions. For example, in case of a balloon guide catheter, the lumen between the inner and outer tubes may be intended for passage of a liquid therethrough. In this case, the size of the flow passage lumen will have an impact on how fast the balloon expands or collapses.
5. Through configuring the outer diameter of the inner tube at the distal end thereof to be smaller than the outer diameter of the inner tube main segment at the proximal end thereof, the catheter comprises a compliance profile increasing from the proximal to distal end, which ensures good delivery performance and accessibility of the catheter.

### BRIEF DESCRIPTION OF THE DRAWINGS

Those of ordinary skill in the art would appreciate that the following drawings are presented merely to enable a better understanding of the present invention rather than limit the scope thereof in any sense. In the drawings,
Fig. 1 is a schematic overview of a catheter according to a preferred embodiment of the present invention in an expanded configuration;
Fig. 2 is a schematic overview of a catheter according to a preferred embodiment of the present invention in a collapsed configuration;
Fig. 3 is a cross-sectional view of a distal portion of a catheter according to a preferred embodiment of the present invention;
Fig. 4 is a cross-sectional view of a distal portion of a catheter according to a preferred embodiment of the present invention;
Fig. 5 is a cross-sectional view of a distal portion of a catheter according to a preferred embodiment of the present invention;
Fig. 6 is a cross-sectional view of a distal portion of a catheter according to a preferred embodiment of the present invention;
Fig. 7 is a cross-sectional view of a distal portion of a catheter according to a preferred embodiment of the present invention;
Fig. 8 is a cross-sectional view of a distal portion of a catheter according to a preferred embodiment of the present invention;
Fig. 9 is a cross-sectional view of a distal portion of a catheter according to a preferred embodiment of the present invention;
Fig. 10 is a schematic overview of a catheter according to a preferred embodiment of the present invention in an expanded configuration;
Fig. 11 is a cross-sectional view of a distal portion of a catheter according to a preferred embodiment of the present invention; and
Fig. 12 is a cross-sectional view of a distal portion of a catheter according to a preferred embodiment of the present invention.

In these figures,
100, a tubular component; 200, an occlusion component; 101, an outer tube; 102, an inner tube; 1011, an outer tube main segment; 1012, a first recess; 1012-1, a first transition section; 1012-2, a first straight section; 1013, an outer-tube distal section; 1013-1, a second transition section; 1013-2, a second straight section; 1014, liquid passage apertures; 1021, an inner tube main segment; 1022, a second recess; 1022-1, a third transition section; 1022-2, a third straight section; 1023, an inner-tube distal section; 1023-1, a fourth transition section; 1023-2, a fourth straight section; 300, a first transition location; and 400, a second transition location.

### DETAILED DESCRIPTION

Objects, advantages and features of the present invention will become more apparent from the following more detailed description of particular embodiments made in conjunction with the accompanying drawings. Note that the figures are provided in a very simplified form not necessarily drawn to exact scale for the only purpose of helping to explain the disclosed embodiments in a more convenient and clearer way. In addition, structures shown in the figures are usually a part of actual structures. In particular, as the figures tend to have distinct emphases, they are often drawn to different scales.

As used herein and in the appended claims, the singular forms "a", "an" and "the" include plural referents unless the context clearly dictates otherwise. As used herein and in the appended claims, the term "or" is employed in the sense including "and/or" unless the context clearly dictates otherwise. Additionally, the terms "proximal" and "distal" are generally used to refer to an end closer to an operator and an end closer to a lesion site in a patient, respectively.

In principle, the present invention is intended to provide a catheter, including a tubular component and a functional component, the tubular component including an outer tube and an inner tube, the outer tube sleeved over the inner tube, and a first lumen formed between the outer and inner tubes, the outer tube including an outer tube main segment and a first recess located at a distal end of the outer tube main segment, the functional component disposed on the tubular component and at least partially connected to the first recess. The functional component may be any component disposed on the catheter to perform a medical function, such as an occlusion, radiopaque, electronic, thrombus removal or other component. The functional component may be connected to the catheter at one end and free at the other end. Alternatively, it may be connected to the catheter at both ends. Still alternatively, it may be overall secured to the catheter. Structural details of the catheter of the present invention will be described in detail below with reference to the following embodiments, in which an occlusion component is described as a representative example of the functional component. Other types of the functional component may have similar relationships with the catheter to that of the occlusion component.

A more detailed description is set forth with reference to the accompanying drawings.

### Embodiment 1

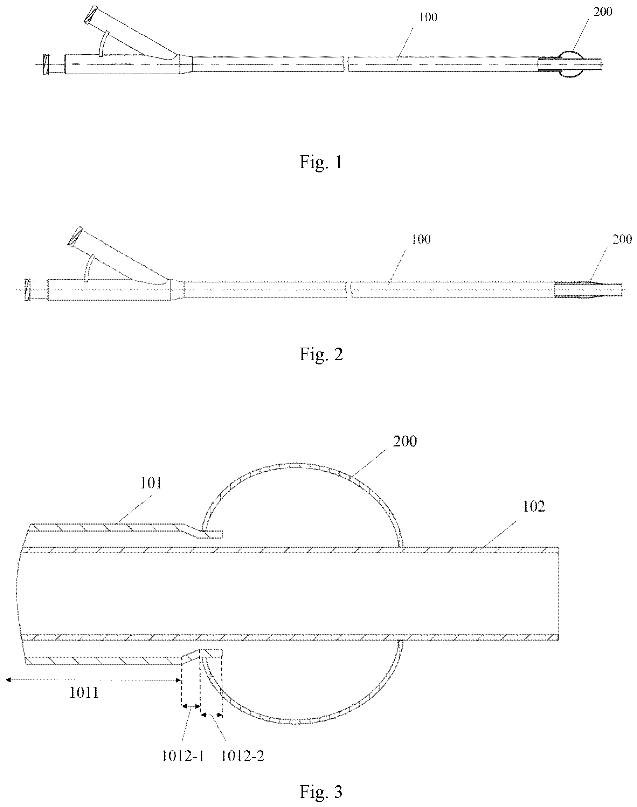

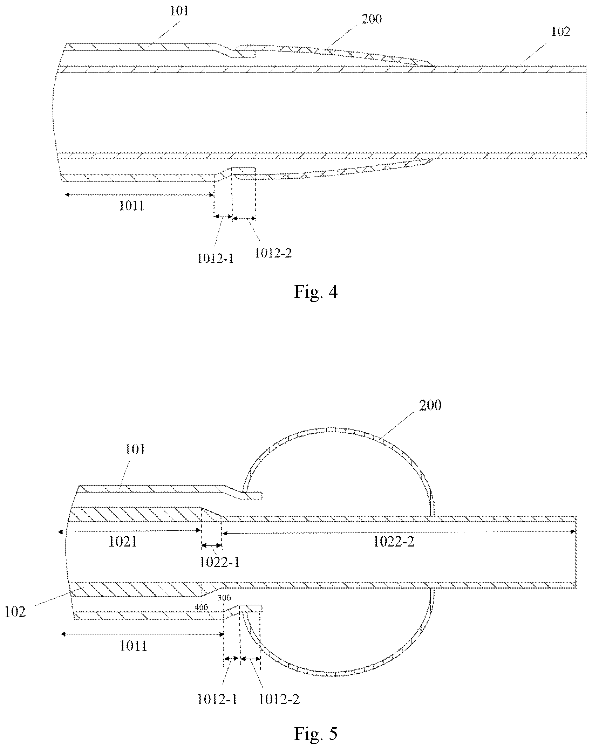

In a first embodiment of the present invention, there is provided a catheter. Figs. 1 and 2 are schematic overviews of this catheter, and Figs. 3 and 4 are cross-sectional views of a distal portion thereof. As shown in Figs. 1 to 4, the catheter of the present invention includes a tubular component 100 and an occlusion component 200. The tubular component 100 includes an outer tube 101 and an inner tube 102. The occlusion component 200 is secured to the tubular component 100. The outer tube 101 is sleeved over the inner tube 102, and a first lumen is formed between the outer tube 101 and the inner tube 102. The occlusion component 200 is configured to comprise an expanded configuration or a collapsed configuration. Figs. 1 and 3 respectively depict a schematic overview and a cross-sectional view of a distal portion of the occlusion component 200 with the occlusion component 200 being in the expanded configuration, and Figs. 2 and 4 respectively show a schematic overview and a cross-sectional view of the distal portion of the occlusion component 200 with the occlusion component 200 being in the collapsed configuration. The occlusion component 200 is switchable between the expanded and collapsed configurations. When in the expanded configuration, the occlusion component 200 is able to block or reduce blood flow in a blood vessel. As shown in Figs. 3 and 4, the outer tube 101 includes an outer tube main segment 1011 and a first recess 1012 located at a distal end of the outer tube main segment 1011. A proximal end of the occlusion component 200 is secured to the first recess 1012 and a distal end of the occlusion component 200 is secured to the inner tube 102. In this way, the occlusion component 200 is secured between the outer tube 101 and the inner tube 102. This can reduce influence of the presence of the occlusion component 200 on an overall outer diameter and compliance of the catheter.

In Embodiment 1, the first recess 1012 is formed by an inward recess in an outer surface of the outer tube 101. In some other embodiments, it may be formed by an outward recess in an inner surface of the outer tube 101.

In Embodiment 1, an outer diameter of the first recess 1012 is smaller than an outer diameter of the outer tube main segment 1011, and an inner diameter of the first recess 1012 is smaller than an inner diameter of the outer tube main segment 1011. A proximal end of the occlusion component 200 is secured to an outer surface of the first recess 1012. In some other embodiments, the outer diameter of the first recess 1012 may be smaller than the outer diameter of the outer tube main segment 1011, and the inner diameter of the first recess 1012 may be equal to the inner diameter of the outer tube main segment 1011, with a proximal end of the occlusion component 200 being secured to the outer surface of the first recess 1012. In some other embodiments, the outer diameter of the first recess 1012 may be equal to the outer diameter of the outer tube main segment 1011, and the inner diameter of the first recess 1012 may be greater than the inner diameter of the outer tube main segment 1011, with a proximal end of the occlusion component 200 being secured to an inner surface of the first recess 1012. In some other embodiments, the outer diameter of the first recess 1012 may be smaller than the outer diameter of the outer tube main segment 1011, and the inner diameter of the first recess 1012 may be greater than the inner diameter of the outer tube main segment 1011, with a proximal end of the occlusion component 200 being secured to the inner or outer surface of the first recess 1012.

In each case, the first recess 1012 has an axial length of 2-20 mm. In Embodiment 1, the axial length of the first recess 1012 is 12 mm. In some other embodiments, the axial length of the first recess 1012 may be 2 mm. In some other embodiments, the axial length of the first recess 1012 may be 5 mm. In some other embodiments, the axial length of the first recess 1012 may be 10 mm. In some other embodiments, the axial length of the first recess 1012 may be 15 mm. In some other embodiments, the axial length of the first recess 1012 may be 20 mm. The recess provided in the outer tube 101 of the catheter can accommodate at least a part of the occlusion component 200, allowing a reduced joint thickness which can partially or completely eliminate the influence of the occlusion component on the catheter's hardness. In this way, it can be ensured that the catheter has sufficient flexibility that allows its smooth advancement through a blood vessel. Moreover, the catheter is allowed to have a reduced overall thickness, which enables the catheter to have a sufficiently large inner cavity but not an excessive outer diameter. Thus, relatively large medical devices can be delivered through the catheter's inner cavity, and the catheter itself can be smoothly advanced within a tortuous blood vessel while less stimulating the wall thereof to access a relatively distal location in the blood vessel.

As shown in Figs. 3 and 4, in Embodiment 1, the first recess 1012 includes a proximal first transition section 1012-1 and a distal first straight section 1012-2. An inner diameter of the first transition section 1012-1 varies from the inner diameter of the outer tube main segment 1011 to an inner diameter of the first straight section 1012-2. Moreover, an outer diameter of the first transition section 1012-1 varies from the outer diameter of the outer tube main segment 1011 to an outer diameter of the first straight section 1012-2. That is, the first transition section 1012-1 is a diameter-varying section where both the inner and outer diameters of the outer tube 101 decrease. In some other embodiments, the outer diameter of the first recess 1012 may be smaller than the outer diameter of the outer tube main segment 1011, and the inner diameter of the first recess 1012 may be equal to the inner diameter of the outer tube main segment 1011. In these cases, the outer diameter of the first transition section 1012-1 varies from the outer diameter of the outer tube main segment 1011 to the outer diameter of the first straight section 1012-2, while the inner diameter of the first transition section 1012-1 is equal to the inner diameter of the outer tube main segment 1011 and the inner diameter of the first recess 1012. Thus, the first transition section 1012-1 is a diameter-varying section where the outer diameter of the outer tube 101 decreases. In some other embodiments, the outer diameter of the first recess 1012 may be equal to the outer diameter of the outer tube main segment 1011, and the inner diameter of the first recess 1012 may be greater than the inner diameter of the outer tube main segment 1011. In these cases, the inner diameter of the first transition section 1012-1 varies from the inner diameter of the outer tube main segment 1011 to the inner diameter of the first straight section 1012-2, while the outer diameter of the first transition section 1012-1 is equal to the outer diameter of the outer tube main segment 1011 and the outer diameter of the first recess 1012. The first transition section 1012-1 is also a diameter-varying section where the inner diameter of the outer tube 101 increases.

As shown in Figs. 3 and 4, in Embodiment 1, each of inner and outer surfaces of the first transition section 1012-1 is inclined at an angle of 45° with respect to an axis of the outer tube main segment 1011 (or of the tubular component 100). In some other embodiments, both the inner and outer surfaces of the first transition section 1012-1 may be inclined at the same angle that is greater than 0 and smaller than or equal to 90° with respect to the axis of the outer tube main segment 1011 (or of the tubular component 100). In some other embodiments, both the inner and outer surfaces of the first transition section 1012-1 may be inclined at the same angle of 60° with respect to the axis of the outer tube main segment 1011 (or of the tubular component 100). In some other embodiments, both the inner and outer surfaces of the first transition section 1012-1 may be inclined at the same angle of 5° with respect to the axis of the outer tube main segment 1011 (or of the tubular component 100). In some other embodiments, both the inner and outer surfaces of the first transition section 1012-1 may be inclined at the same angle of 85° with respect to the axis of the outer tube main segment 1011 (or of the tubular component 100). In some other embodiments, both the inner and outer surfaces of the first transition section 1012-1 may be perpendicular to the axis of the outer tube main segment 1011 (or of the tubular component 100). In some other embodiments, the inner surface of the first transition section 1012-1 may be parallel to the axis of the outer tube main segment 1011 (or of the tubular component 100), while the outer surface of the first transition section 1012-1 may be inclined at an angle with respect to the axis of the outer tube main segment 1011 (or of the tubular component 100), which may be greater than 0° and smaller than 90°, such as 5°, 15°, 30°, 40°, 45°, 60°, 75° or 85°. In some other embodiments, the inner surface of the first transition section 1012-1 may be parallel to the axis of the outer tube main segment 1011 (or of the tubular component 100), while the outer surface of the first transition section 1012-1 may be perpendicular to the outer tube main segment 1011 (or of the tubular component 100). In some other embodiments, the outer surface of the first transition section 1012-1 may be parallel to the axis of the outer tube main segment 1011 (or of the tubular component 100), while the inner surface of the first transition section 1012-1 may be inclined at an angle with respect to the axis of the outer tube main segment 1011 (or of the tubular component 100), which may be any value greater than 0° and smaller than or equal to 90°, such as 5°, 15°, 30°, 40°, 45°, 60°, 75° or 85°. In some other embodiments, the outer surface of the first transition section 1012-1 may be parallel to the axis of the outer tube main segment 1011 (or of the tubular component 100), while the inner surface of the first transition section 1012-1 may be perpendicular to the axis of the outer tube main segment 1011 (or of the tubular component 100). In each case, the first transition section 1012-1 has an axial length of 0-10 mm. In Embodiment 1, the axial length of the first transition section 1012-1 is 4 mm. In some other embodiments, the axial length of the first transition section 1012-1 may be 0 mm. In some other embodiments, the axial length of the first transition section 1012-1 may be 3 mm. In some other embodiments, the axial length of the first transition section 1012-1 may be 5 mm. In some other embodiments, the axial length of the first transition section 1012-1 may be 8 mm. In some other embodiments, the axial length of the first transition section 1012-1 may be 10 mm.

In each case, the outer diameter of the outer tube main segment 1011 is 1.0 mm to 3.7 mm, the outer diameter of the first straight section 1012-2 is 0.7 mm to 3.5 mm, and a ratio of the outer diameter of the first straight section 1012-2 to the outer diameter of the outer tube main segment 1011 is 0.7-1.0. In Embodiment 1, the outer diameter of the outer tube main segment 1011 is 2.8 mm, the outer diameter of the first straight section 1012-2 is 2.6 mm, and the ratio of the outer diameter of the first straight section 1012-2 to the outer diameter of the outer tube main segment 1011 is 0.928. In some other embodiments, the outer diameter of the outer tube main segment 1011 may be 3.7 mm, the outer diameter of the first straight section 1012-2 may be 2.8 mm, and the ratio of the outer diameter of the first straight section 1012-2 to the outer diameter of the outer tube main segment 1011 may be 0.757. In some other embodiments, the outer diameter of the outer tube main segment 1011 may be 3.5 mm, the outer diameter of the first straight section may be 3.5 mm, and the ratio of the outer diameter of the first straight section 1012-2 to the outer diameter of the outer tube main segment 1011 may be 1.0. In some other embodiments, the outer diameter of the outer tube main segment 1011 may be 1.0 mm, the outer diameter of the first straight section 1012-2 may be 0.7 mm, and the ratio of the outer diameter of the first straight section 1012-2 to the outer diameter of the outer tube main segment 1011 may be 0.7.

In each case, the catheter has an overall length of 80-160 cm. In Embodiment 1, the overall length of the catheter is 130 cm. In some other embodiments, the overall length of the catheter may be 80 cm. In some other embodiments, the overall length of the catheter may be 160 cm. In some other embodiments, the overall length of the catheter may be 115 cm. In some other embodiments, the overall length of the catheter may be 110 cm. In some other embodiments, the overall length of the catheter may be 140 cm. In some other embodiments, the overall length of the catheter may be 150 cm.

In Embodiment 1, the occlusion component 200 is a polymer film. The first lumen is configured for passage or suction of a liquid therethrough, which can cause a switch of the occlusion component 200 between the expanded and collapsed configurations. The liquid that can be passed or sucked through the first lumen may be, for example, a contrast fluid, a physiological saline solution or the like. Filling the first lumen with the liquid can bring the occlusion component 200 into the expanded configuration. Emptying the first lumen can bring the occlusion component 200 into the collapsed configuration. In Embodiment 1, the polymer film has a thickness of 0.10 mm. In some other embodiments, the thickness of the polymer film may be 0.05 mm to 0.15 mm, such as 0.05 mm, 0.08 mm, 0.12 mm or 0.15 mm. In Embodiment 1, the polymer film is made of silicone. In some other embodiments, the polymer film may be made of polyurethane (PU). In some other embodiments, the polymer film may be made of latex. In some other embodiments, the polymer film may be made of polyethylene (PE). In some other embodiments, the polymer film may be made of polytrafluoroethylene (PTFE). In some other embodiments, the polymer film may be made of expanded PTFE (ePTFE). In some other embodiments, the polymer film may be made of PU and PE mixed at a ratio of 2: 1. In some other embodiments, the polymer film may be made of PTFE and ePTFE mixed at a ratio of 1: 1. In some other embodiments, the polymer film may be made of silicone, PU and PE mixed at a ratio of 1: 1: 1. In Embodiment 1, a proximal end of the occlusion component 200 is connected to the first straight section 1012-2, for example, by gluing, bonding or fusion. In some other embodiments, a proximal end of the occlusion component 200 may be connected to the first transition section 1012-1, for example, by gluing, bonding or fusion.

In Embodiment 1, the inner tube 102 is a triple-layered structure consisting of an innermost first polymer layer, an intermediate reinforcing layer and an outermost second polymer layer. The first polymer layer is made of PTFE, and the reinforcing layer is a structure braided from stainless steel filaments. The second polymer layer is composed of axially spliced polyether block amide (PEBA), nylon, PU, PE and polyolefin elastomer (POE). The outer tube 101 consists of a single polymer layer made of PEBA. In some other embodiments, both the inner tube 102 and the outer tube 101 may be triple-layered structures each consisting of an innermost first polymer layer, an intermediate reinforcing layer and an outermost second polymer layer. In some other embodiments, the inner tube 102 may be a single-layered polymer structure, and the outer tube 101 may be a triple-layered structure. In some other embodiments, the inner tube 102 may be a triple-layered structure, and the outer tube 101 may be a double-layered polymer structure. In some other embodiments, the reinforcing layer(s) in the inner tube 102 and/or the outer tube 101 may each be a structure consisting of spirally wound filaments. In some other embodiments, the reinforcing layer(s) in the inner tube 102 and/or the outer tube 101 may each be a cut tube. In some other embodiments, the reinforcing layer(s) in the inner tube 102 and/or the outer tube 101 may each consist of both a structure braided from filament and a structure consisting of spirally wound filaments. In some other embodiments, the reinforcing layer(s) in the inner tube 102 and/or the outer tube 101 may each consisting of a structure braided from filament and a cut tube. In some other embodiments, the reinforcing layer(s) in the inner tube 102 and/or the outer tube 101 may each consist of a cut tube and a structure consisting of spirally wound filaments. In some other embodiments, the reinforcing layer(s) in the inner tube 102 and/or the outer tube 101 may each be made of a material including a nickel-titanium (NiTi) alloy. In some other embodiments, the reinforcing layer(s) in the inner tube 102 and/or the outer tube 101 may be each made of a material including a cobalt-chromium (CoCr) alloy. In some other embodiments, the reinforcing layer(s) in the inner tube 102 and/or the outer tube 101 may each be made of a material including a polymer. In some other embodiments, the reinforcing layer(s) in the inner tube 102 and/or the outer tube 101 may each be made of both a NiTi alloy and stainless steel. In some other embodiments, the reinforcing layer(s) in the inner tube 102 and/or the outer tube 101 may each be made of both a NiTi alloy and a polymer.

In Embodiment 1, the catheter includes a first radiopaque ring disposed at a head portion of the catheter and a second radiopaque ring disposed at a location of the inner tube 102 that is adapted to the location of the occlusion component 200.

In Embodiment 1, the inner tube 102 of the catheter forms a second lumen therein, which has a constant inner diameter across its entire length. In each case, the second lumen has an inner diameter of 0.1 mm to 3.0 mm, and the outer diameter of the outer tube main segment 1011 is 0.5 mm to 3.7 mm. In Embodiment 1, the inner diameter of the second lumen is 2.3 mm, the outer diameter of the outer tube main segment 1011 is 2.8 mm, and a ratio of the inner diameter of the second lumen to the outer diameter of the outer tube main segment 1011 is 0.821. In some other embodiments, the inner diameter of the second lumen may be 0.1 mm, the outer diameter of the outer tube main segment 1011 may be 0.5 mm, and the ratio of the inner diameter of the second lumen to the outer diameter of the outer tube main segment 1011 may be 0.2. In some other embodiments, the inner diameter of the second lumen may be 3.0 mm, the outer diameter of the outer tube main segment 1011 may be 3.6 mm, and the ratio of the inner diameter of the second lumen to the outer diameter of the outer tube main segment 1011 may be 0.833. In some other embodiments, the inner diameter of the second lumen may be 2.7 mm, the outer diameter of the outer tube main segment 1011 may be 3.0 mm, and the ratio of the inner diameter of the second lumen to the outer diameter of the outer tube main segment 1011 may be 0.9. In some other embodiments, the inner diameter of the second lumen may be 2.5 mm, the outer diameter of the outer tube main segment 1011 may be 3.7 mm, and the ratio of the inner diameter of the second lumen to the outer diameter of the outer tube main segment 1011 may be 0.676, In Embodiment 1, the second lumen is configured for passage of a medical device therethrough.

In Embodiment 1, there are angled transitions between the outer tube main segment 1011 and the first recess 1012, and between the first transition section 1012-1 and the first straight section 1012-2. In some other embodiments, there may be smooth curved transition(s) between the outer tube main segment 1011 and first recess 1012, and/or between the first transition section 1012-1 and the first straight section 1012-2. In Embodiment 1, the first straight section 1012-2 has a flat, smooth surface. In some other embodiments, the first straight section 1012-2 may provide on its surface with convexities and concavities, grooves or curved portions, while having constant inner and outer diameters across its entire length.

### Embodiment 2

In a second embodiment of the present invention, there is provided a catheter. Fig. 5 is a cross-sectional view of a distal portion of this catheter with an occlusion component 200 of the catheter being in an expanded configuration. As shown in Fig. 5, the catheter of Embodiment 2 has an overall structure substantially similar to that of the catheter of Embodiment 1, and further description thereof is, therefore, deemed unnecessary. Differing from that of Embodiment 1, an inner tube 102 in the catheter of Embodiment 2 includes an inner tube main segment 1021 and a second recess 1022 located at a distal end of the inner tube main segment 1021. An outer diameter of the second recess 1022 is smaller than an outer diameter of the inner tube main segment 1021, and a proximal end of the occlusion component 200 is secured to a first recess 1012 and a distal end of the occlusion component 200 is secured to the second recess 1022. The recess provided in the inner tube 102 of the catheter can accommodate at least a part of the occlusion component 200, allowing a reduced joint thickness which can partially or completely eliminate the influence of the occlusion component on the catheter's hardness. In this way, it can be ensured that the catheter has sufficient flexibility that allows its smooth advancement through a blood vessel. Moreover, the recess provided in the inner tube 102 makes the catheter more flexible at the distal end, additionally facilitating delivery of the catheter in a blood vessel.

In each case, the second recess 1022 has an axial length of 2-60 mm. In Embodiment 2, the axial length of the second recess 1022 is 30 mm. In some other embodiments, the axial length of the second recess 1022 may be 2 mm. In some other embodiments, the axial length of the second recess 1022 may be 10 mm. In some other embodiments, the axial length of the second recess 1022 may be 25 mm. In some other embodiments, the axial length of the second recess 1022 may be 45 mm. In some other embodiments, the axial length of the second recess 1022 may be 60 mm.

As shown in Fig. 5, the second recess 1022 includes a proximal third transition section 1022-1 and a distal third straight section 1022-2. An outer diameter of the third transition section 1022-1 varies from the outer diameter of the inner tube main segment 1021 to an outer diameter of the third straight section 1022-2. The third transition section 1022-1 is a diameter-varying section where the outer diameter of the inner tube 102 decreases. Similar to the first transition section 1012-1 of the first recess 1012, an outer surface of the third transition section 1022-1 is inclined at an angle of 10° with respect to an axis of the inner tube main segment 1021 (or of the tubular component 100). In some other embodiments, the outer surface of the third transition section 1022-1 may be inclined at an angle of 5°with respect to the axis of the inner tube main segment 1021 (or of the tubular component 100). In some other embodiments, the outer surface of the third transition section 1022-1 may be inclined at an angle of 15°with respect to the axis of the inner tube main segment 1021 (or of the tubular component 100). In some other embodiments, the outer surface of the third transition section 1022-1 may be inclined at an angle of 25°with respect to the axis of the inner tube main segment 1021 (or of the tubular component 100). In some other embodiments, the outer surface of the third transition section 1022-1 may be inclined at an angle of 20°with respect to the axis of the inner tube main segment 1021 (or of the tubular component 100). In some other embodiments, the outer surface of the third transition section 1022-1 may be perpendicular to the axis of the inner tube main segment 1021 (or of the tubular component 100). In some embodiments, an inner surface of the third transition section 1022-1 is parallel to the axis of the inner tube main segment 1021 (or of the tubular component 100), and the outer surface of the third transition section 1022-1 is inclined at an angle with respect to the axis of the inner tube main segment 1021 (or of the tubular component 100), which may be any value in the range of 0-90°, such as 5°, 15°, 30°, 40°, 45°, 60°, 75° or 85°. In some other embodiments, the inner surface of the third transition section 1022-1 may be parallel to the axis of the inner tube main segment 1021 (or of the tubular component 100), and the outer surface of the third transition section 1022-1 may be perpendicular to the axis of the inner tube main segment 1021 (or of the tubular component 100). In some other embodiments, the inner surface of the third transition section 1022-1 may be inclined at an angle with respect to the axis of the inner tube main segment 1021 (or of the tubular component 100), which may be value in the range of 0-90°, such as 5°, 15°, 30°, 40°, 45°, 60°, 75° or 85°. In some other embodiments, the inner surface of the third transition section 1022-1 may be perpendicular to the axis of the inner tube main segment 1021 (or of the tubular component 100). In each case, the third transition section 1022-1 has an axial length of 0-10 mm. In Embodiment 2, the axial length of the third transition section 1022-1 is 5 mm. In some other embodiments, the axial length of the third transition section 1022-1 may be 0 mm. In some other embodiments, the axial length of the third transition section 1022-1 may be 3 mm. In some other embodiments, the axial length of the third transition section 1022-1 may be 5 mm. In some other embodiments, the axial length of the third transition section 1022-1 may be 8 mm. In some other embodiments, the axial length of the third transition section 1022-1 may be 10 mm.

In some embodiments, the outer diameter of the inner tube main segment 1021 is 0.5 mm to 3.2 mm, the outer diameter of the third straight section 1022-2 is 0.3 mm to 3.2 mm, and a ratio of the outer diameter of the third straight section 1022-2 to the outer diameter of an outer tube main segment 1011 is 0.6-1.0. In Embodiment 2, the outer diameter of the inner tube main segment 1021 is 2.8 mm, the outer diameter of the third straight section 1022-2 is 2.4 mm, and the ratio of the outer diameter of the third straight section 1022-2 to the outer diameter of the outer tube main segment 1011 is 0.857. In some other embodiments, the outer diameter of the inner tube main segment 1021 may be 3.2 mm, the outer diameter of the third straight section 1022-2 may be 3.2 mm, and a ratio of the outer diameter of the third straight section 1022-2 to the outer diameter of the inner tube main segment 1021 may be 1.0. In some other embodiments, the outer diameter of the inner tube main segment 1021 may be 0.5 mm, the outer diameter of the third straight section 1022-2 may be 0.3 mm, and the ratio of the outer diameter of the third straight section 1022-2 to the outer diameter of the inner tube main segment 1021 may be 0.6. In some other embodiments, the outer diameter of the inner tube main segment 1021 may be 1.0 mm, the outer diameter of the third straight section 1022-2 may be 0.8 mm, and the ratio of the outer diameter of the third straight section 1022-2 to the outer diameter of the inner tube main segment 1021 may be 0.8. In some other embodiments, the outer diameter of the inner tube main segment 1021 may be 2.0 mm, the outer diameter of the third straight section 1022-2 may be 1.8 mm, and the ratio of the outer diameter of the third straight section 1022-2 to the outer diameter of the inner tube main segment 1021 may be 0.9.

As shown in Fig. 5, in the catheter of Embodiment 2, the outer tube main segment 1011 comprises a first transition location 300 at its most distal end (i.e., the first transition location 300 is located at an interface between the outer tube main segment 1011 and the first recess 1012). In Embodiment 2, the first transition location 300 is a position where the outer and inner diameters of the outer tube 101 in the catheter start varying. In some other embodiments, the first transition location 300 is a position where the outer diameter and/or inner diameter of the outer tube 101 in the catheter may start varying. The inner tube main segment 1021 in the catheter forms a second transition location 400 at its most distal end (i.e., the second transition location 400 is located at an interface between the inner tube main segment 1021 and the second recess 1022). In Embodiment 2, the second transition location 400 is a position where the outer diameter of the inner tube starts varying. In some other embodiments, the second transition location 400 may be a position where both the outer and inner diameters of the inner tube 102 in the catheter start varying. In the catheter, the first transition location 300 may be a cross-section having the same cross-sectional shape as at the most distal end of the outer tube main segment 1011, and the second transition location 400 may be a cross-section having the same cross-sectional shape as at the most distal end of the inner tube main segment 1021. Hereinafter, for ease of description, they are referred to as the "first transition location 300" and the "second transition location 400", respectively. In Embodiment 2, a projection of the first transition location 300 on the axis of the tubular component 100 is distal with respect to a projection of the second transition location 400 on the tubular component 100. Moreover, the outer surface of the third transition section 1022-1 is inclined at a first angle with respect to the axis of the tubular component 100, and the inner surface of the first transition section 1012-1 is inclined at a second angle with respect to the axis of the tubular component 100. The first angle is greater than or equal to the second angle. In Embodiment 2, the inner diameter of the outer tube 101 in the catheter starts decreasing at the first transition location 300, and the outer diameter of the inner tube 102 in the catheter starts decreasing at the second transition location. Through configuring the projection of the first transition location 300 on the axis of the tubular component 100 to be distal with respect to the projection of the second transition location 400 on the axis of the tubular component 100, and through configuring the angle between the third transition section 1022-1 and the axis of the catheter to be greater than the angle between the first transition section 1012-1 and the axis of the catheter, it can be ensured that the size of the first lumen will not shrink much due to the reduced inner diameter of the outer tube 101. When the first lumen is used to allow passage or suction of a liquid therethrough, this can ensure sufficient passage or suction efficiency.

In Embodiment 2, both the inner tube 102 and the outer tube 101 are triple-layered structures each consisting of an innermost first polymer layer, an intermediate reinforcing layer and an outermost second polymer layer. The first polymer layer in the inner tube 102 is made of PTFE and POE. The reinforcing layer in the inner tube 102 is a structure consisting of spirally wound NiTi alloy filaments. The second polymer layer in the inner tube 102 is a structure consisting of axially spliced PEBA, nylon, PU, PTFE, PE, PEBA mixed with an additive resulting in a lower friction coefficient and POE. The first polymer in the outer tube 101 is made of PTFE. The reinforcing layer in the outer tube 101 is a structure braided from polymer filaments. The second polymer layer in the outer tube 101 is a structure consisting of axially spliced PEBA, nylon, PU, PE and POE.

In Embodiment 2, the catheter includes a first radiopaque ring disposed at an end of the catheter.

In Embodiment 2, there are angled transitions between the inner tube main segment 1021 and the second recess 1022, and between the third transition section 1022-1 and the third straight section 1022-2. In some other embodiments, there may be smooth curved transition(s) between the inner tube main segment 1021 and the second recess 1022, and/or between the third transition section 1022-1 and the third straight section 1022-2. In Embodiment 2, the third straight section 1022-2 has a flat, smooth surface. In some other embodiments, the third straight section 1022-2 may provide on its surface with convexities and concavities, grooves or curved portions, while having constant inner and outer diameters across its entire length.

### Embodiment 3

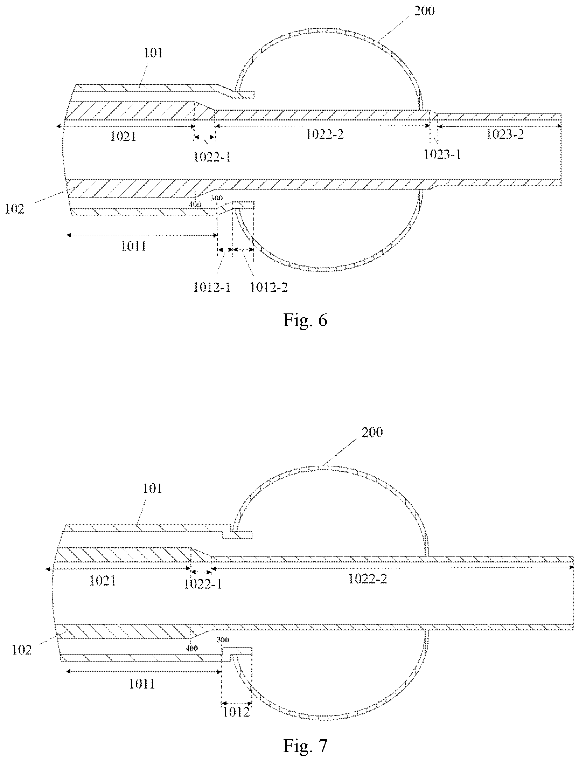

In a third embodiment of the present invention, there is provided a catheter. Fig. 6 is a cross-sectional view of a distal portion of this catheter with an occlusion component 200 of the catheter being in an expanded configuration. As shown in Fig. 6, the catheter of Embodiment 3 has an overall structure substantially similar to that of the catheter of Embodiment 2, and further description thereof is, therefore, deemed unnecessary. Differing from that of Embodiment 2, an inner tube 102 in the catheter of Embodiment 3 includes an inner tube main segment 1021, a second recess 1022 and an inner-tube distal section 1023. The second recess 1022 is disposed at a distal end of the inner tube main segment 1021 and has an outer diameter smaller than an outer diameter of the inner tube main segment 1021. A proximal end of the occlusion component 200 is secured to a first recess 1012 and a distal end of the occlusion component 200 is secured to the second recess 1022. The inner-tube distal section 1023 is disposed at a distal end of the second recess 1022 and has an outer diameter smaller than the outer diameter of the second recess 1022. The inner-tube distal section 1023 locates at a head portion of the catheter. The presence of the inner-tube distal section 1023 makes compliance of the catheter gradually increase from its proximal to distal end, ensuring satisfactory delivery performance and accessibility of the catheter.

As shown in Fig. 6, the inner-tube distal section 1023 includes a proximal fourth transition section 1023-1 and a distal fourth straight section 1023-2. An outer diameter of the fourth transition section 1023-1 varies from an outer diameter of a third straight section 1022-2 to an outer diameter of the fourth straight section. The fourth transition section 1023-1 is a diameter-varying section where an outer diameter of the inner tube 102 decreases. In some embodiments, the outer diameter of the fourth straight section 1023-2 is 0.2 mm to 3.1 mm. In Embodiment 3, the outer diameter of the fourth straight section 1023-2 is 2.0 mm. In some other embodiments, the outer diameter of the fourth straight section 1023-2 may be 0.2 mm. In some other embodiments, the outer diameter of the fourth straight section 1023-2 may be 1.5 mm. In some other embodiments, the outer diameter of the fourth straight section 1023-2 may be 3.1 mm. In Embodiment 3, the outer diameter of the fourth straight section 1023-2 is smaller than the outer diameter of the second recess 1022, and an inner diameter of the fourth straight section 1023-2 is equal to an inner diameter of the second recess 1022. In some other embodiments, the outer diameter of the fourth straight section 1023-2 may be smaller than the outer diameter of the second recess 1022, and the inner diameter of the fourth straight section 1023-2 may be greater than the inner diameter of the second recess 1022. Through providing the fourth straight section 1023-2 at the distal end of the inner tube 102, which has a smaller outer diameter than the inner tube 102 at the proximal end thereof, the hardness of the catheter at the distal end thereof can be additionally reduced, enhancing the ability of the catheter to pass through a blood vessel, reducing the risk of the distal end of the catheter causing damage to the blood vessel and improving its accessibility.

In some other embodiments, the inner-tube distal section 1023 may include 2-10 transition sections and straight sections. The transition sections may alternate with the straight sections, resulting in a gradually decreasing outer diameter profile of the inner-tube distal section 1023. The outer diameter of the inner-tube distal section 1023 may gradually decrease from 3 mm at the proximal end to 0.6 mm at the distal end. In some other embodiments, the inner-tube distal section 1023 may include 5 alternating transition and straight sections, and the outer diameter of the inner-tube distal section 1023 may decrease from 2.7 mm at the proximal end to 0.9 mm at the distal end. In some other embodiments, the inner-tube distal section 1023 may include 10 alternating transition and straight sections, and the outer diameter of the inner-tube distal section 1023 may decrease from 3.0 mm at the proximal end to 0.6 mm at the distal end. In some other embodiments, the inner-tube distal section 1023 may include 2 alternating transition and straight sections, and the outer diameter of the inner-tube distal section 1023 may decrease from 2.4 mm at the proximal end to 1.65 mm at the distal end. In some other embodiments, the inner-tube distal section 1023 may be a taped tubular structure with a gradually decreasing outer diameter. In some embodiments, the outer diameter of the inner-tube distal section 1023 may decrease from 3 mm at the proximal end to 0.6 mm at the distal end. In some embodiments, the outer diameter of the inner-tube distal section 1023 may decrease from 2.5 mm at the proximal end to 0.6 mm at the distal end. In some embodiments, the outer diameter of the inner-tube distal section 1023 may decrease from 2 mm at the proximal end to 0.9 mm at the distal end.

In Embodiment 3, a projection of a first transition location 300 on an axis of a tubular component 100 is distal with respect to a projection of a second transition location 400 on the axis of the tubular component 100.

In Embodiment 3, the inner tube 102 is a triple-layered structure consisting of an innermost first polymer layer, an immediate reinforcing layer and an outermost second polymer layer. The outer tube 101 is a double-layered structure consisting of an outer polymer layer and an inner reinforcing layer. The reinforcing layer of the outer tube 101 is provided by a cut tube.

In Embodiment 3, the catheter includes a second radiopaque ring disposed at a location of the inner tube 102 that is adapted to the location of the occlusion component 200.

In Embodiment 3, there are angled transitions between the second recess 1022 and the inner-tube distal section 1023, and between the fourth transition section 1023-1 and the fourth straight section 1023-2. In some other embodiments, there may be smooth curved transition(s) between the second recess 1022 and the inner-tube distal section 1023, and/or between the fourth transition section 1023-1 and the fourth straight section 1023-2. In Embodiment 3, the fourth straight section 1023-2 has a flat, smooth surface. In some other embodiments, the fourth straight section 1023-2 may provide on its surface with convexities and concavities, grooves or curved portions, while having constant inner and outer diameters across its entire length.

### Embodiment 4

In a fourth embodiment of the present invention, there is provided a catheter. Fig. 7 is a cross-sectional view of a distal portion of this catheter with an occlusion component 200 of the catheter being in an expanded configuration. As shown in Fig. 7, the catheter of Embodiment 4 has an overall structure substantially similar to that of the catheter of Embodiment 2, and further description thereof is, therefore, deemed unnecessary. Differing from that of Embodiment 2, in the catheter of Embodiment 4, a first transition section 1012-1 in a first recess 1012 is a diameter-varying section perpendicular to an axis of a tubular component 100. That is, both outer and inner surfaces of the first transition section 1012-1 form an angle of 90° with respect to the axis of the tubular component 100. An axial length of the first transition section 1012-1, i.e., a wall thickness of the tube there, is 0.1 mm, and an axial length of the first recess 1012 is 5 mm.

In Embodiment 4, a projection of a first transition location 300 on the axis of the tubular component 100 is distal with respect to a projection of a second transition location 400 on the axis of the tubular component 100.

In Embodiment 4, the inner tube 102 is a triple-layered structure consisting of an innermost first polymer layer, an immediate reinforcing layer and an outermost second polymer layer. The outer tube 101 is a double-layered structure consisting of an outer polymer layer and an inner reinforcing layer. The reinforcing layer of the outer tube 101 is provided by a cut tube.

In Embodiment 4, the catheter includes a first radiopaque ring disposed at a head portion of the catheter and second and third radiopaque rings disposed at locations of the inner tube 102 that are adapted to the location of the occlusion component 200. Specifically, the second radiopaque ring may be disposed at a distal end of the occlusion component 200, and the third radiopaque ring may be disposed at a proximal end of the occlusion component 200.

### Embodiment 5

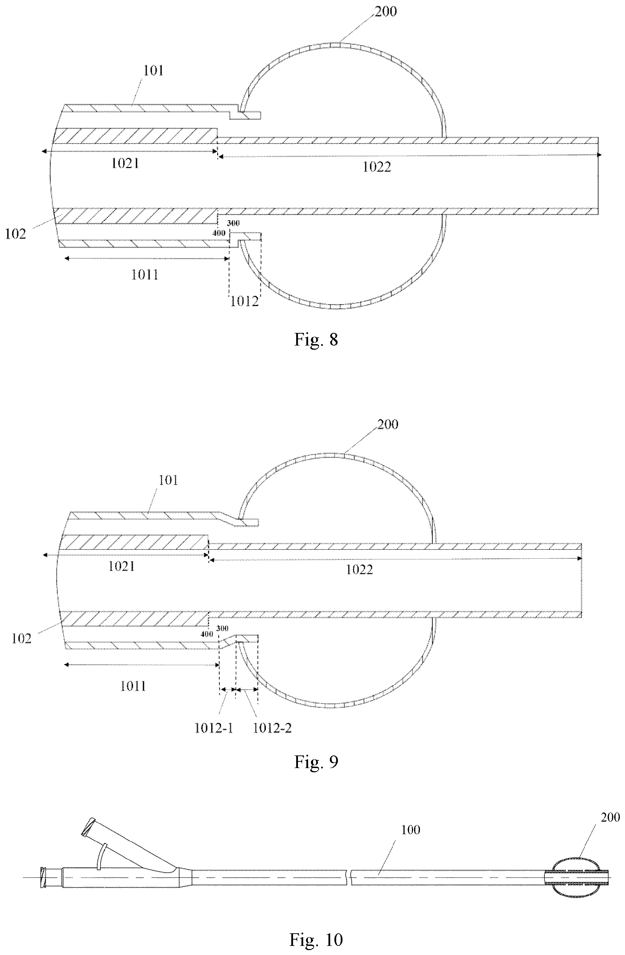

In a fifth embodiment of the present invention, there is provided a catheter. Fig. 8 is a cross-sectional view of a distal portion of this catheter with an occlusion component 200 of the catheter being in an expanded configuration. As shown in Fig. 8, the catheter of Embodiment 5 has an overall structure substantially similar to that of the catheter of Embodiment 4, and further description thereof is, therefore, deemed unnecessary. Differing from that of Embodiment 4, in the catheter of Embodiment 5, a third transition section 1022-1 in a second recess 1012 is a diameter-varying section perpendicular to an axis of a tubular component 100. That is, both outer and inner surfaces of the third transition section 1022-1 form an angle of 90° with the axis of the tubular component 100. An axial length of a first transition section 1013-1 is 0.5 mm, and an axial length of a first recess 1012 is 8 mm. The first transition section 1012-1 in the first recess 1012 is a diameter-varying section perpendicular to the axis of the tubular component 100. That is, an outer surface of the first transition section 1012-1 forms an angle of 90° with the axis of the tubular component 100. An axial length of the third transition section 1022-1 may be 0 mm, and an axial length of the second recess 1022 may be 20 mm. In Embodiment 5, an inner diameter of the second recess 1022 is equal to an inner diameter of an inner tube main segment 1021, and a thickness of the inner tube 102 at a distal end thereof is smaller than a thickness of the inner tube main segment 1021. In some other embodiments, an outer diameter of the second recess 1022 may be smaller than an outer diameter of the inner tube main segment 1021, the inner diameter of the second recess 1022 may be greater than the inner diameter of the inner tube main segment 1021, and the thickness of the inner tube at the distal end thereof may be smaller than the thickness of the inner tube main segment 1021. In some other embodiments, the outer diameter of the second recess 1022 may be smaller than the outer diameter of the inner tube main segment 1021, and the inner diameter of the second recess 1022 may be smaller than the inner diameter of the inner tube main segment 1021.

In Embodiment 5, a projection of a first transition location 300 on the axis of the tubular component 100 is distal with respect to a projection of a second transition location 400 on the axis of the tubular component 100.

In Embodiment 5, the inner tube 102 is a double-layered structure consisting of an inner first polymer layer and an outer second polymer layer. The outer tube 101 is a single-layered polymer structure.

In Embodiment 5, the catheter includes second and third radiopaque rings disposed at locations of the inner tube 102 that are adapted to the location of the occlusion component 200. Specifically, the second radiopaque ring may be disposed at a distal end of the occlusion component 200, and the third radiopaque ring may be disposed at a proximal end of the occlusion component 200.

As shown in Fig. 9, in some other embodiments, the third transition section 1022-1 in the second recess 1012 is a diameter-varying section perpendicular to the axis of the tubular component 100. That is, both the outer and inner surfaces of the third transition section 1022-1 form an angle of 90° with the axis of the tubular component 100. The axial length of the third transition section 1022-1 may be 0.05 mm, and the axial length of the second recess 1022 may be 8 mm. Additionally, both the inner and outer surfaces of the first transition section 1012-1 in the first recess 1012 may be inclined at angles with respect to the axis of the tubular component 100, and the angles may be both 40°.

### Embodiment 6

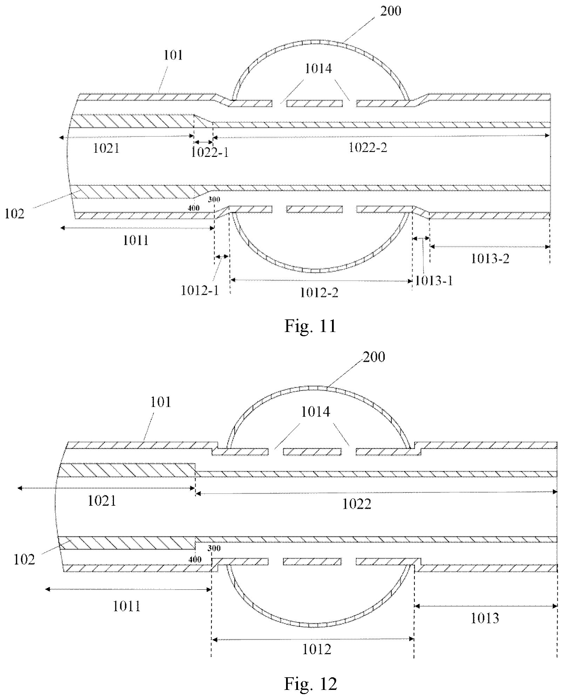

In a sixth embodiment of the present invention, there is provided a catheter. Fig. 10 is a schematic overview of this catheter, and Fig. 11 is a cross-sectional view of a distal portion of the catheter. As shown in Figs. 10 and 11, the catheter of the present invention includes a tubular component 100 and an occlusion component 200. The tubular component 100 includes an outer tube 101 and an inner tube 102. The occlusion component 200 is secured to the outer tube 101 of the tubular component 100. The outer tube 101 is sleeved over the inner tube 102, and a first lumen is formed between the outer tube 101 and the inner tube 102. The occlusion component 200 is configured to comprise an expanded configuration or a collapsed configuration. The occlusion component 200 is switchable between the expanded and collapsed configurations. When in the expanded configuration, the occlusion component 200 is able to block or reduce blood flow in a blood vessel. As shown in Fig. 11, the outer tube 101 includes an outer tube main segment 1011 and a first recess 1012 located at a distal end of the outer tube main segment 1011. The occlusion component 200 is secured to the first recess 1012. Liquid passage apertures 1014 are formed in the first recess 1012. The first lumen is configured for passage or suction of a liquid therethrough, which can cause a switch of the occlusion component 200 between the expanded and collapsed configurations. Dilating the first lumen with a liquid can bring the occlusion component 200 into the expanded configuration. Emptying the first lumen can bring the occlusion component 200 into the collapsed configuration. The liquid passage apertures 1014 are configured for passage of a liquid therethrough from the first lumen into the occlusion component 200 to bring the occlusion component 200 into the expanded configuration. The liquid may be sucked out from the occlusion component 200 to bring the occlusion component 200 into the collapsed configuration. The first recess 1012 has been described above in detail in Embodiment 1 to 5 and, therefore, needs not be described in further detail here.

As shown in Fig. 11, in Embodiment 6, the outer tube 101 further includes an outer-tube distal section 1013 disposed at a distal end of the first recess 1012 and having an outer diameter at a proximal end of the outer-tube distal section that is greater than an outer diameter of the first recess 1012 at the distal end of the outer-tube distal section. The outer-tube distal section 1013 is fixedly coupled at a distal end thereof to the inner tube 102. The outer-tube distal section 1013 includes a proximal second transition section 1013-1 and a distal second straight section 1013-2. An inner diameter of the second straight section 1013-2 is greater than an inner diameter of the first straight section 1012-2, and an outer diameter of the second straight section 1013-2 is greater than an outer diameter of the first straight section 1012-2. Inner and outer diameters of the second transition section 1013-1 gradually vary from the inner and outer diameters of the first straight section 1012-2 to the inner and outer diameters of the second straight section 1013-2 from the proximal end to the distal end, respectively. Te second transition section 1013-1 is a diameter-varying section where inner and outer diameters of the outer tube 101 both increase. In some other embodiments, the inner diameter of the second straight section 1013-2 may be equal to the inner diameter of the first straight section 1012-2, and the outer diameter of the second straight section 1013-2 may be greater than the outer diameter of the first straight section 1012-2. Additionally, the outer diameter of the second transition section 1013-1 may gradually vary from the outer diameter of the first straight section 1012-2 to the outer diameter of the second straight section 1013-2 from the proximal end to the distal end, with the inner diameter of the second transition section 1013-1 remaining constant. Te second transition section 1013-1 is a diameter-varying section where the outer diameter of the outer tube 101 increases. The outer-tube distal section 1013 and the first recess may be connected to each other to form a V-shaped, frame-like, curved, polygonal, irregular or other recess on outer tube. In Embodiment 6, the occlusion component 200 is connected at both proximal and distal end thereof to the first straight section 1012-2, optionally by gluing, bonding or fusion. In some other embodiments, a proximal end of the occlusion component 200 may be connected to the first transition section 1012-1 and/or a distal end thereof to the second transition section 1013-1, optionally by gluing, bonding or fusion.

In Embodiment 6, inner and outer surfaces of the second transition section 1013-1 are inclined both at the same angle of 60° with respect to an axis of the tubular component 100. In some other embodiments, the inner and outer surfaces of the second transition section 1013-1 may be inclined both at the same or different angles with respect to the axis of the tubular component 100, which may each be any value in the range of 0-90°, such as 5°, 15°, 30°, 40°, 45°, 60°, 75° or 85°. In some other embodiments, the inner and outer surfaces of the second transition section 1013-1 may be both perpendicular to the axis of the tubular component 100. In some other embodiments, the inner surface of the second transition section 1013-1 may be parallel to the axis of the tubular component 100, and the outer surface of the second transition section 1013-1 may be included at an angle with respect to the axis of the tubular component 100, which may be any value in the range of 0-90°, such as 5°, 15°, 30°, 40°, 45°, 60°, 75° or 85°. In some other embodiments, the inner surface of the second transition section 1013-1 may be parallel to the axis of the tubular component 100, and the outer surface of the second transition section 1013-1 may be perpendicular to the axis of the tubular component 100. In each case, an axial length of the second transition section 1013-1 is 0-10 mm. In Embodiment 6, the axial length of the second transition section 1013-1 is 5 mm. In some other embodiments, the axial length of the second transition section 1013-1 may be 0 mm. In some other embodiments, the axial length of the second transition section 1013-1 may be 3 mm. In some other embodiments, the axial length of the second transition section 1013-1 may be 8 mm. In some other embodiments, the axial length of the second transition section 1013-1 may be 10 mm. In each case, an axial length of the outer-tube distal section 1013 is 1-15 mm. In Embodiment 6, the axial length of the outer-tube distal section 1013 is 10 mm. In some other embodiments, the axial length of the outer-tube distal section 1013 may be 1 mm. In some other embodiments, the axial length of the outer-tube distal section 1013 may be 8 mm. In some other embodiments, the axial length of the outer-tube distal section 1013 may be 12 mm. In some other embodiments, the axial length of the outer-tube distal section 1013 may be 15 mm.

In each case, the outer diameter of the second straight section 1013-2 is 1.0-3.7 mm. In Embodiment 6, the outer diameter of the second straight section 1013-2 is 2.8 mm. In some other embodiments, the outer diameter of the second straight section 1013-2 may be 1.0 mm. In some other embodiments, the outer diameter of the second straight section 1013-2 may be 2.0 mm. In some other embodiments, the outer diameter of the second straight section 1013-2 may be 3.0 mm. In some other embodiments, the outer diameter of the second straight section 1013-2 may be 3.7 mm.

In Embodiment 6, the outer-tube distal section 1013 is connected to the inner tube 102 (not shown) so that a distal end of the first lumen is closed. As a result, when a liquid is flowing through the first lumen, it will not leak from the distal end of the catheter, thereby enabling expansion and collapse of the occlusion component 200. To this end, there may be a diameter-varying section (not shown) at the distal end of the outer-tube distal section 1013, which may have outer diameter gradually decreasing from its proximal to distal end and can be connected to the inner tube 102. The outer-tube distal section 1013 may be connected to the inner tube 102 either at the most distal end of the inner tube 102, or at another location thereof.

As shown in Fig. 11, the inner tube 102 in the catheter of Embodiment 6 may include a proximal inner tube main segment 1021 and a distal second recess 1022. The second recess 1022 is located at a distal end of the inner tube main segment 1021 and has an outer diameter smaller than an outer diameter of the inner tube main segment 1021. The second recess 1022 has been described above in detail in Embodiment 2 to 5 and, therefore, needs not be described in further detail here. In some other embodiments, the inner tube 102 in the catheter may be a straight tube with constant inner and outer diameters across its entire length. In some other embodiments, the inner tube 102 of the catheter may include an inner tube main segment 1021, a second recess 1022 located at a distal end of the inner tube main segment 1021 and an inner-tube distal section 1023 located at a distal end of the second recess 1022. An outer diameter of the second recess 1022 may be smaller than an outer diameter of the inner tube main segment 1021, and an outer diameter of the inner-tube distal section 1023 may be smaller than the outer diameter of the second recess 1022.

In Embodiment6, a projection of a first transition location 300 on the axis of the tubular component 100 is distal with respect to a projection of a second transition location 400 on the axis of the tubular component 100.

In Embodiment 6, the inner tube 102 is a triple-layered structure consisting of an innermost first polymer layer, an immediate reinforcing layer and an outermost second polymer layer. The outer tube 101 is a double-layered structure consisting of an outer layer that is a polymer layer and an inner layer that is a polymer layer.

In Embodiment 6, the catheter includes a first radiopaque ring sleeved over the inner tube and located at a head portion of the catheter. The catheter also includes a second radiopaque ring sleeved over the inner tube 102 at a location that is adapted to the location of the occlusion component 200.

As shown in Fig. 12, in some other embodiments, in the outer-tube distal section 1013, the second transition section 1013-1 may be a diameter-varying section perpendicular to the axis of the tubular component 100. That is, the outer and inner surfaces of the second transition section 1013-1 may both form an angle of 90° with the axis of the tubular component 100.

In Embodiment 6, there are angled transitions between the first recess 1012 and the outer-tube distal section 1013, and between the second transition section 1013-1 and the second straight section 1013-2. In some other embodiments, there may be smooth curved transition(s) between the first recess 1012 and the outer-tube distal section 1013, and/or between the second transition section 1013-1 and the second straight section 1013-2. In Embodiment 6, the second straight section 1013-2 has a flat, smooth surface. In some other embodiments, the second straight section 1013-2 may provide on its surface with convexities and concavities, grooves or curved portions, while having constant inner and outer diameters across its entire length.

### Embodiment 7

In a seventh embodiment of the present invention, there is provided a catheter including: a first recess 1012 having a taped outer surface with a gradually decreasing outer diameter; and a proximal end of an occlusion component 200 secured to the tapered surface of the first recess 1012. That is, the first recess 1012 includes only a first transition section 1012-1 but not a first straight section 1012-2.

In some other embodiments, a distal end of an outer tube may have a first beveled surface, which forms an outer surface of the first recess 1012. Moreover, a proximal end of an occlusion component 200 may have a second surface, which mates with, and is fixedly connected to, the first beveled surface. That is, the first recess 1012 includes only a first transition section 1012-1 but not a first straight section 1012-2. Further, the beveled surface of the occlusion component 200 located at the proximal end may be sloped at the same angle as the beveled surface of the outer tube at the distal end so that they can mate with and be fixedly connected to each other.

The foregoing description presents some preferred embodiments of the present invention and is not intended to limit the scope of the present invention in any way. Any and all changes and modifications made by those of ordinary skill in the art in light of the above teachings without departing from the spirit of the present invention are intended to be embraced in the scope as defined by the appended claims.

## Claims

1. A catheter, comprising a tubular component and a functional component,
wherein the tubular component comprises an outer tube and an inner tube,
wherein the outer tube is sleeved over the inner tube, and a first lumen is formed between the outer and inner tubes, wherein the outer tube comprises an outer tube main segment and a first recess, and wherein the first recess is located at a distal end of the outer tube main segment, and
wherein the functional component is disposed on the tubular component and is at least partially connected to the first recess.

2. The catheter of claim 1, wherein the functional component is at least one of an occlusion component, a radiopaque component, an electronic element and a thrombus removal element.

3. The catheter of claim 1, wherein the first recess is formed by an inward recess in an outer surface of the outer tube.

4. The catheter of claim 3, wherein an inner diameter of the first recess is smaller than or equal to an inner diameter of the outer tube main segment.

5. The catheter of claim 1, wherein the first recess is formed by an outward recess in an inner surface of the outer tube.

6. The catheter of claim 5, wherein an outer diameter of the first recess is smaller than or equal to an outer diameter of the outer tube main segment.

7. The catheter of claim 1, wherein the first recess has an axial length of 2-30 mm.

8. The catheter of claim 1, wherein the first recess comprises a first transition section and a first straight section from a proximal end to a distal end thereof, and wherein the first transition section is a diameter-varying section where at least one of an inner diameter and an outer diameter of the outer tube increases or decreases.

9. The catheter of claim 8, wherein the first transition section has an axial length of 0 mm to 10 mm.

10. The catheter of claim 8, wherein an inner surface and/or an outer surface of the first transition section is/are each inclined at an angle of 0°-90° with respect to an axis of the outer tube main segment.

11. The catheter of claim 10, wherein the inner and outer surfaces of the first transition section are inclined at a same angle with respect to the axis of the outer tube main segment.

12. The catheter of claim 8, wherein a ratio of an outer diameter of the first straight section to an outer diameter of the outer tube main segment is 0.7-1.0.

13. The catheter of claim 12, wherein the outer diameter of the outer tube main segment is 1.0 mm to 3.7 mm, and wherein the outer diameter of the first straight section is 0.7 mm to 3.5 mm.

14. The catheter of claim 1, wherein the outer tube further comprises an outer-tube distal section located at a distal end of the first recess, wherein an outer diameter of the outer-tube distal section at a proximal end thereof is greater than an outer diameter of the first recess at the distal end thereof, and wherein the outer-tube distal section is fixedly connected to the inner tube at a distal location.

15. The catheter of claim 14, wherein the outer-tube distal section comprises a second transition section and a second straight section from a proximal end to a distal end thereof, and wherein the second transition section is a diameter-varying section where at least one of an inner diameter and an outer diameter of the outer tube increases or decreases.

16. The catheter of any one of claims 1 to 15, wherein the inner tube comprises an inner tube main segment and a second recess, wherein the second recess is located at a distal end of the inner tube main segment, and wherein the second recess is formed by an inward recess in an outer surface of the inner tube.

17. The catheter of claim 16, wherein the second recess comprises a third transition section and a third straight section from a proximal end to a distal end thereof, and wherein the third transition section is a diameter-varying section where an outer diameter of the inner tube decreases.

18. The catheter of claim 16, wherein the second recess has an axial length of 2-60 mm.

19. The catheter of claim 17, wherein an outer surface of the third transition section is inclined at an angle of 0°-90° with respect to an axis of the inner tube main segment, and wherein the third transition section has an axial length of 0-10 mm.

20. The catheter of claim 17, wherein a ratio of an outer diameter of the third straight section to an outer diameter of the inner tube main segment is greater than or equal to 0.6 and is smaller than 1.0.

21. The catheter of claim 20, wherein the outer diameter of the inner tube main segment is 0.5 mm to 3.2 mm, and wherein the outer diameter of the third straight section is greater than or equal to 0.3 mm and is smaller than 3.2 mm.

22. The catheter of claim 17, wherein a most distal end of the outer tube main segment forms a first transition location, and wherein a most distal end of the inner tube main segment forms a second transition location, and wherein a projection of the first transition location on the axis of the tubular component is distal with respect to a projection of the second transition location on the axis of the tubular component;
wherein the first recess comprises a first transition section and a first straight section from a proximal end to a distal thereof; and
wherein an outer surface of the third transition section is inclined at a first angle with respect to the axis of the tubular component, wherein an inner surface of the first transition section is inclined at a second angle with respect to the axis of the tubular component, and wherein the first angle is greater than or equal to the second angle.

23. The catheter of claim 16, wherein the inner tube further comprises an inner-tube distal section located at a distal end of the second recess.

24. The catheter of claim 23, wherein the inner-tube distal section has an axial length of 1-500 mm.

25. The catheter of claim 23, wherein an outer diameter of the inner-tube distal section is smaller than an outer diameter of the second recess, and wherein the inner-tube distal section is disposed at a head portion of the catheter.

26. The catheter of claim 23, wherein the inner-tube distal section comprises a fourth transition section and a fourth straight section from a proximal end to a distal end thereof, and wherein the fourth transition section is a diameter-varying section where an outer diameter of the inner tube decreases.

27. The catheter of claim 26, wherein the fourth straight section has an outer diameter of 0.2 mm to 3.1 mm.

28. The catheter of claim 2, wherein the functional component is an occlusion component, and wherein a proximal end of the occlusion component is secured to the first recess.

29. The catheter of claim 28, wherein the occlusion component is a polymer film, wherein the occlusion component is in an expanded configuration when the first lumen is filled with a liquid, and wherein the occlusion component is in a collapsed configuration when the first lumen is in a vacuum.

30. The catheter of claim 28, wherein a distal end of the occlusion component is fixedly connected to the inner tube, or
wherein the occlusion component is disposed on the first recess and each of a proximal end and the distal end of the occlusion component is fixedly connected to the outer tube, wherein a distal end of the outer tube is connected to the inner tube, and wherein the first recess is provided with liquid passage apertures for dilation of the occlusion component with a liquid.

31. The catheter of claim 29, wherein the polymer film has a thickness of 0.05 mm to 0.15 mm, and/or wherein the polymer film is made of any one of silicone, polyurethane, latex, polyethylene, polytrafluoroethylene and expanded polytrafluoroethylene, or of a mixture thereof.

32. The catheter of claim 1, wherein the inner tube comprises an inner tube main segment and a second recess, wherein the second recess is disposed at a distal end of the inner tube main segment, and wherein the second recess has an outer diameter smaller than an outer diameter of the inner tube main segment, wherein the functional component is an occlusion component, and wherein a proximal end of the occlusion component is fixedly connected to the first recess and a distal end of the occlusion component is fixedly connected the second recess.

33. The catheter of claim 1, wherein each of the inner and outer tubes comprises at least one polymer layer made of one or more of polyether block amide, nylon, polyurethane, polytrafluoroethylene, polyethylene and polyolefin elastomer.

34. The catheter of claim 33, wherein the outer tube and/or the inner tube further comprise(s) reinforcing layer(s), wherein the reinforcing layer is a structure braided from filaments, a structure consisting of spirally wound filaments, a cut tube or a combination thereof, and wherein the reinforcing layer is made of stainless steel, a nickel-titanium alloy, a cobalt-chromium alloy or a polymer.

35. The catheter of claim 34, wherein the outer tube and/or the inner tube is/are triple-layered structure(s) each consisting of an innermost first polymer layer, an intermediate reinforcing layer and an outermost second polymer layer.

36. The catheter of claim 1, further comprising: a first radiopaque ring disposed at a head portion of the catheter; and/or
a second radiopaque ring disposed at a location on the inner tube, wherein the second radiopaque ring is adapted to a location of the functional component.

37. The catheter of claim 1, wherein the inner tube is provided with a second lumen having a constant inner diameter across an entire length thereof.

38. The catheter of claim 37, wherein a ratio of the inner diameter of the second lumen to an outer diameter of the outer tube main segment is 0.2-0.9.

39. The catheter of claim 38, wherein the inner diameter of the second lumen is 0.1 mm to 3.0 mm, and wherein the outer diameter of the outer tube main segment is 0.5 mm to 3.7 mm.

40. The catheter of claim 1, wherein the first recess has a tapered outer surface with a gradually decreasing outer diameter, and wherein a proximal end of the functional component is secured to the tapered surface of the first recess.

41. The catheter of claim 40, wherein a distal end face of the outer tube is a first beveled surface forming the outer surface of the first recess, wherein the functional component forms at a proximal end thereof a second beveled surface mating with and fixedly connected to the first beveled surface.

42. An occlusion catheter, comprising a tubular component and an occlusion component,
wherein the tubular component comprises an outer tube and an inner tube,
wherein the outer tube is sleeved over the inner tube, and a first lumen is formed between the outer and inner tubes, wherein the outer tube comprises an outer tube main segment and a first recess, and wherein the first recess is located at a distal end of the outer tube main segment, and
wherein the occlusion component is disposed on the tubular component and is at least partially connected to the first recess.

43. The occlusion catheter of claim 42, wherein the occlusion component is a polymer film, wherein the occlusion component is in an expanded configuration when the first lumen is filled with a liquid, and wherein the occlusion component is in a collapsed configuration when the first lumen is in a vacuum.
